(19) Europäisches Patentamt
European Patent Office
Office européen des brevets

(11) Veröffentlichungsnummer : **0 051 738**
**B1**

(12) # EUROPÄISCHE PATENTSCHRIFT

(45) Veröffentlichungstag der Patentschrift : **25.07.84**

(21) Anmeldenummer : **81107893.0**

(22) Anmeldetag : **03.10.81**

(51) Int. Cl.$^3$ : **C 09 K 3/34**, G 02 F 1/133, C 07 C121/64, C 07 C 25/18, C 07 C 43/225

(54) **Fluorhaltige Cyclohexylbiphenylderivate, diese enthaltende Dielektrika und elektrooptisches Anzeigeelement.**

(30) Priorität : **10.11.80 DE 3042391**

(43) Veröffentlichungstag der Anmeldung : **19.05.82 Patentblatt 82/20**

(45) Bekanntmachung des Hinweises auf die Patenterteilung : **25.07.84 Patentblatt 84/30**

(84) Benannte Vertragsstaaten : **AT CH DE FR GB IT LI NL**

(56) Entgegenhaltungen :
**DE-B- 2 701 591**
**ANGEWANDTE CHEMIE, INTERNATIONAL EDITION IN ENGLISH, Band 16, Februar 1977, Seite 100 Weinheim, DE. R. EIDENSCHINK et al.: "Substituted Phenylcyclohexanes - A New Class of Liquid-Crystalline Compounds"**
**AUgenchemie Int. (Engl.) Bd. 16 Seite 100 (1977)**
**Die Akte enthält technische Angaben, die nach dem Eingang der Anmeldung eingereicht wurden und die nicht in dieser Patentschrift enthalten sind.**

(73) Patentinhaber : **Merck Patent Gesellschaft mit beschränkter Haftung**
**Frankfurter Strasse 250**
**D-6100 Darmstadt (DE)**

(72) Erfinder : **Eidenschink, Rudolf, Dr.**
**Erlenweg 17**
**D-6110 Dieburg (DE)**
Erfinder : **Pohl, Ludwig, Dr.**
**Niebergallweg 5**
**D-6100 Darmstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 738 B1

Jouve, 18, rue St-Denis, 75001 Paris, France

**Beschreibung**

Für elektrooptische Anzeigeelemente werden in großem Umfang die Eigenschaften nematischer oder nematisch-cholesterischer flüssigkristalliner Materialien ausgenutzt, ihre optischen Eigenschaften wie Lichtabsorption, Lichtstreuung, Doppelbrechnung, Reflexionsvermögen oder Farbe unter dem Einfluß elektrischer Felder signifikant zu verändern. Die Funktion deratiger Anzeigeelemente beruht dabei beispielsweise auf den Phänomenen der dynamischen Streuung, der Deformation aufgerichteter Phasen, dem Schadt-Helfrich-Effekt in der verdrillten Zelle oder dem cholesterisch-nematischen Phasenübergang.

Für die technische Anwendung dieser Effekte in elektronischen Bauelementen werden flüssigkristalline Dielektrika benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Dielektrika eine flüssigkristalline Mesophase im Temperaturbereich von mindestens + 10 °C bis + 50 °C, bevorzugt von 0 °C bis 60 °C, und eine möglichst niedrige Viskosität bei Raumtemperatur, die vorzugsweise nicht mehr als $70 \cdot 10^{-3}$ Pa.s. betragen soll, gefordert. Schließlich dürfen sie im Bereich des sichtbaren Lichtes keine Eigenabsorption aufweisen, d. h. sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS-2 139 638 beschriebenen p,p'-disubstituierten Benzoesäurephenylester und die in der DE-OS-2 636 684 beschriebenen p,p'-disubstituierten Phenylcyclohexanderivate. In beiden genannten Verbindungsklassen wie auch in anderen bekannten Reihen von Verbindungen mit flüssigkristalliner Mesophase gibt es keine Einzelverbindungen, die in dem geforderten Temperaturbereich von 10 °C bis 60 °C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klärpunkt mit einer anderen mit deutlich höherem Schmelz- und Klärpunkt. Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Trotzdem bereitet die Herstellung optimaler Dielektrika immer wieder Schwierigkeiten, da die Komponenten mit den hohen Schmelz- und Klärpunkten den Gemischen häufig auch eine hohe Viskosität verleihen. Dadurch werden die Schaltzeiten der damit hergestellten elektrooptischen Anzeigeelemente in unverwünschter Weise verlängert. Ferner treten oft Probleme dadurch auf, daß die Löslichkeit der verschiedenen Komponenten ineinander, insbesondere bei Raumtemperatur oder tieferen Temperaturen nur sehr begrenzt ist.

Die erfindungsgemäßen Verbindungen sind den Verbindungen des Standes der Technick überlegen, da sie einen hohen Klärpunkt, eine niedrige Viskosität und einen breiten nematischen Bereich in sich vereinen.

Der Erfindung liegt die Aufgabe zugrunde, flüssigkristalline Dielektrika herzustellen, die eine nematische Phase im geforderten Temperaturbereich aufweisen und in Flüssigkristallzellen bei Raumtemperatur ausreichend kurze Schaltzeiten ermöglichen.

Es wurde non gefunden, daß die Cyclohexylbiphenylderivate der Formel (I)

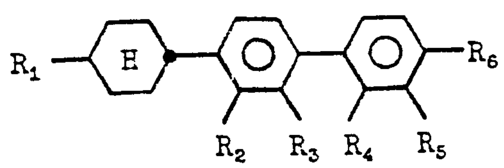

(I)

worin

$R_1$ Alkyl mit 1-12 C-Atomen,

$R_6$ Alkyl oder Alkoxy mit 1-12 C-Atomen, CN oder Fluor, und

$R_2$, $R_3$, $R_4$ und $R_5$ Wasserstoff oder Fluor bedeuten, mit der Maßgabe, daß mindestens einer, jedoch nicht mehr als zwei der Reste $R_2$, $R_3$, $R_4$, $R_5$ und $R_6$ Fluor bedeuten,

vorzüglich als Komponenten flüssigkristalliner Dielektrika geeignet sind. Dabei besitzen diese Verbindungen einen außerordentlichen breiten Anwendungsbereich :

in Abhängigkeit von der Auswahl der Substituenten können die Verbindungen der Formel (I) sowohl als Basismaterialien dienen, aus denen flüssigkristalline Dielektrika zum überwiegenden Teil bestehen, es können aber auch Verbindungen der Formel (I) in geringeren Anteilen von beispielsweise 2 bis 45 Gewichtsprozent flüssigkristallinen Basismaterialien aus anderen Verbindungsklassen zugesetzt werden, um so Dielektrika mit einer verbreiterten flüssigkristallinen Mesophase herzustellen oder die Größe der dielektrischen Anisotropie eines solchen Dielektrikums zu beeinflussen.

Durch geeignete Auswahl der Substituenten $R_1$ bis $R_6$ lassen sich mit den Verbindungen der Formel (I) sowohl Dielektrika mit ausgeprägter positiver dielektrischer Anisotropie zur Verwendung in Anzeigeelementen auf der Basis der verdrillten nematischen Zelle oder des cholesterisch-nematischen Phasenübergangs herstellen, es können aber auch Dielektrika mit von Null nur wenig verschiedener oder auch mit negativer dielektrischer Anisotropie hergestellt werden, die in Anzeigeelementen auf der Basis der dynamischen Streuung oder der Deformation aufgerichteter Phasen (DAP-Effekt) verwendet werden.

Die Verbindungen der Formel (I) sind in reinem Zustand farblos, und bilden niederviskose nematische Mesophasen in einem erstaunlich breiten und für die elektrooptische Verwendung günstig gelegenen Temperaturbereich.

Gegenstand der Erfindung sind somit die Cyclohexylbiphenylderivate der Formel (I) und ihre Verwendung als Komponenten flüssigkristalliner Dielektrika. Gegenstand der Erfindung sind weiterhin flüssigkristalline Dielektriak mit einem Gehalt an mindestens einem Cyclohexylbiphenylderivat der Formel (I) sowie elektrooptische Anzeigeelemente auf der Basis einer Flüssigkristallzelle, die ein derartiges flüssigkristallines Dielektrikum enthalten.

Die erfindungsgemäßen Cyclohexylbiphenylderivate umfassen insbesondere die 4-(trans-4-Alkylcyclohexyl)-4'-fluorbiphenyle der Formel (Ia),

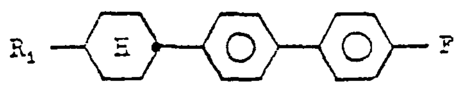

(Ia)

die 4-(trans-4-Alkylcyclohexyl)-3'-fluor-4'-($R_7$)-biphenyle der Formel (Ib),

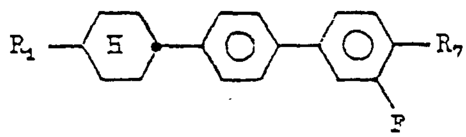

(Ib)

die 4-(trans-4-Alkylcyclohexyl)-3',4'-difluorbiphenyle der Formel (Ic),

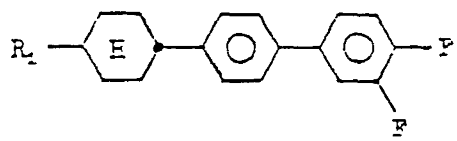

(Ic)

die 4-(trans-4-Alkylcyclohexyl)-2',3'-difluor-4'-($R_7$)-biphenyle der Formel (Id)

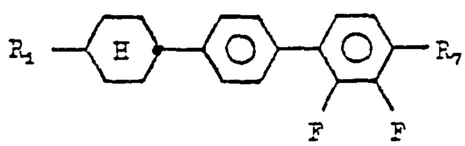

(Id)

die 2,3-Difluor-4-(trans-4-alkylcyclohexyl)-4'-($R_7$)-biphenyle der Formel (Ie)

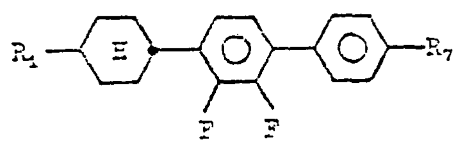

(Ie)

sowie die 2-Fluor-4-(trans-4-alkylcyclohexyl)-4'-($R_7$)-biphenyle der Formel (If),

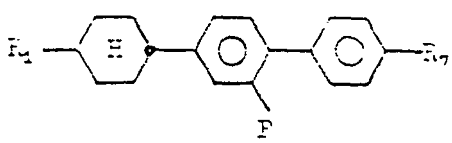

(If)

wobei in diesen Teilformeln $R_7$ Alkyl oder Alkoxy mit 1-12 C-Atomen oder CN bedeutet und $R_1$ die bei der Formel (I) angegebene Bedeutung besitzt. Die trans-Stellung der Substituenten in den 1- und 4-Positionen des Cyclohexanrings ist in den Formelbildern durch einen verstärkten schwarzen Punkt auf der rechten Seite des Ringes kenntlich gemacht. Die nicht von den Teilformeln (Ia) bis (If) umfaßten Verbindungen der Formel (I) besitzen zwar die gleichen vorteilhaften Eigenschaften wie die der herausgehobenen Gruppen, jedoch ist ihre Herstellung schwieriger und daher weniger wirtschaftlich ; die Verbindungen der Teilformeln (Ia) bis (If) sind daher bevorzugt.

Unter den Verbindungen der Formel (I) haben die mit den Teilformeln (Ia) bis (Ic) eine positive dielektrische Anisotropie ; dagegen weisen die Verbindungen der Teilformeln (Id) bis (If) eine negative dielektrische Anisotropie oder Werte um Null auf, wenn $R_7$ Alkyl oder Alkoxy bedeutet, und eine verminderte positive dielektrische Anisotropie, wenn $R_7$ CN ist.

In den Verbindungen der Formel (I) kann Alkylrest $R_1$ sowie der Alkyl- bzw. Alkoxyrest $R_6$ geradkettig oder verzweigt sein. Wenn er geradkettig ist, also Methyl, Ethyl, n-Propyl, n-Butyl, n-Pentyl, n-Hexyl, n-Heptyl, n-Octyl, n-Nonyl, n-Decyl, n-Undecyl oder n-Dodecyl, bedeutet, besitzen die dadurch charakterisierten Verbindungen in der Regel höhere Klärpunkte als die mit verzweigten Flügelgruppen $R_1$ und/oder $R_6$. Deswegen enthält gewöhnlich höchstens eine der Flügelgruppen $R_1$ und $R_6$ eine verzweigte Kohlenstoffkette. Verbindungen der Formel (I) mit einer verzweigten Flügelgruppe $R_1$ oder $R_6$ sind gelegentlich wegen einer besseren Löslichkeit in den üblichen flüssigkristallinen Basismaterialien von Bedeutung, insbesondere aber als chirale Dotierstoffe, wenn sie durch die Kettenverzweigung optische Aktivität besitzen. Solche verzweigten Flügelgruppen enthalten nicht mehr als eine Kettenverzweigung. Bevorzugte verzweigte Kohlenwasserstoffreste sind die, in denen sich an einer längeren Kohlenstoffkette in 1-, 2- oder 3-Stellung eine Methyl- oder Ethylgruppe befindet, beispielsweise 2-Methylpropyl, 2-Methylbutyl, 3-Methylbutyl, 2-Methylpentyl, 3-Methylpentyl, 2-Ethylhexyl oder 1-Methylhexyl. Wenn $R_6$ Alkyl oder Alkoxy bedeutet, können die Flügelgruppen $R_1$ und $R_6$ zusammen bis zu 24 Kohlenstoffatome enthalten. Im Rahmen der vorliegenden Erfindung sind darunter diejenigen bevorzugt, in denen $R_1$ und $R_6$ zusammen 3 bis 14, insbesondere von 4 bis zu 12 Kohlenstoffatomen enthalten.

Die erfindungsgemäßen Verbindungen werden in für derartige Substanzen üblicher Weise hergestellt. So werden die 4-(trans-4-Alkylcyclohexyl)-4'-fluorbiphenyle der Formel (Ia) erhalten, indem die bekannten in 4'-Position unsubstituierten 4-(trans-4-Alkylcyclohexyl)-biphenyle zu den entsprechenden 4'-Nitroverbindungen nitriert werden, die zu den 4'-Aminoverbindungen reduziert und in an sich bekannter Weise nach Schiemann-Balz in die 4'-Fluorverbindungen überführt werden. Zur Herstellung der 3',4'-Difluorverbindungen der Formel (Ic) wird ein 4-(trans-4-Alkylcyclohexyl)-4'-aminobiphenyl zunächst acetyliert, dann zum 3'-Nitro-4'-acetamidobiphenylderivat nitriert, das dann durch Reduktion und Hydrolyse in das 3',4'-Diaminobiphenylderivat überführt wird. Aus diesem wird dann wieder durch Diazotierung, Umsetzung mit Fluoborat und thermische Zersetzung das 3',4'-Difluorbiphenylderivat erhalten.

Die Verbindungen der Formel (I), worin $R_6$ eine Alkyl- oder Alkoxygruppe ist, und von den Resten $R_2$ bis $R_5$ einer oder zwei Fluor bedeuten, werden analog aus den entsprechend substituierten Mono- oder Dinitroverbindungen hergestellt. Diese Ausgangsmaterialien werden durch Nitrierung der 4-(trans-4-Alkylcyclohexyl)-4'-(alkyl- bzw. alkoxy)-biphenyle erhalten. Dabei kann durch geeignete Wahl der an sich aus der Literatur bekannten Nitrierungsbedingungen, z. B. Art und Konzentration des nitrierenden Agens, Lösungsmittel, Temperatur, Reaktionsdauer und/oder Katalysator, die Isomerenverteilung in dem entstehenden Gemisch von Nitrierungsprodukten in Richtung auf die hauptsächlich gewünschten Produkte beeinflußt werden. Aus den erhaltenen Isomerengemischen können diese dann in üblicher Weise abgetrennt werden, zum Beispiel mit chromatografischen Verfahren. Die Reduktion der Nitroverbindungen zu den Aminoverbindungen wird nach Standardmethoden durchgeführt, zum Beispiel durch katalytische Hydrierung, durch Behandlung mit wäßrigem Dithionit oder mit Zinn-(II)-chlorid und Salzsäure. Die Schiemann-Balz-Synthese wird gleichfalls in an sich bekannter Weise durchgeführt, zum Beispiel nach einer der in « Organic Reactions », Band 5 (1949), Seiten 192-228 beschriebenen Verfahrensvarianten.

Die erfindungsgemäßen Dielektrika bestehen aus 2 bis 15, vorzugsweise 3 bis 12 Komponenten, darunter mindestens einem fluorhaltigen Cyclohexylbiphenylderivat der Formel (I). Die anderen Bestandteile werden ausgewählt aus den nematischen oder nematogenen Substanzen aus den Klassen der Azoxybenzole, Benzylidenaniline, Biphenyle, Terphenyle, Phenyl- oder Cyclohexylbenzoate, Cyclohexancarbonsäure-phenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, ggf. halogenierte Stilbene, Benzylphenylether, Tolane und substituierten Zimtsäuren. Die wichtigsten als Bestandteile derartiger flüssigkristalliner Dielektrika in Frage kommenden Verbindungen lassen sich durch die Formel (II) charakterisieren.

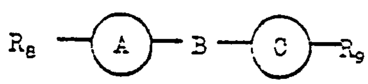
(II)

worin A und C je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

B —CH=CH— —N(O)=N—
—CH=CX— —CH=N(O)—
—C≡C— $—CH_2—CH_2—$
—CO—O— $—CH_2—O—$
—CO—S— $—CH_2—S—$
—N=N—
—CH=N—

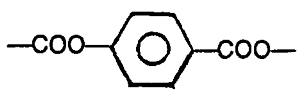

oder eine C—C— Einfachbindung,

X Halogen, vorzugsweise Chlor, oder —CN, und $R_8$ und $R_9$ Alkyl, Alkoxy, Alkanoyloxy oder Alkoxycarbonyloxy mit bis zu 18, vorzugsweise bis zu 8 Kohlenstoffatomen, oder einer dieser Reste auch —CN, —NC, $—NO_2$, $—CF_3$, F, Cl oder Br bedeuten. Bei den meisten dieser Verbindungen sind $R_8$ und $R_9$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrike enthalten in der Regel mindestens 30, vorzugsweise 50-99, insbesondere 60-98 Gewichtsteile der Verbindungen der Formel (I) und (II). Hiervon entfallen bevorzugt mindestens 5 Gewichtsteile, meist auch 10 oder mehr Gewichtsteile auf eine oder mehrere Verbindungen der Formel (I). Es werden von der Erfindung auch solche flüssigkristallinen Dielektrika umfaßt, denen beispielsweise zu Dotierungszwecken nur weniger als 5 Gewichtsteile, zum Beispiel 0,1 bis 3 Gewichtsteile einer oder mehrerer Verbindungen der Formel (I) zugesetzt worden sind. Andererseits können die Verbindungen der Formel (I) bis zu 50 Gewichtsprozent der erfindungsgemäßen Dielektrika ausmachen. Vorzugsweise enthalten die flüssigkristallinen Dielektrika nach der Erfindung 10 bis 30 Gewichtsprozent einer oder mehrerer Verbindungen der Formel (I).

Die Herstellung der erfindungsgemäßen Dielektrika erfolgt in an sich üblicher Weise. In der Regel wird die gewünschte Menge der in geringerer Menge verwendeten Komponenten in der den Hauptbestandteil ausmachenden Komponente gelöst, zweckmäßig bei erhöhter Temperatur. Wenn dabei eine Temperatur oberhalb des Klärpunkts des Hauptbestandteils gewählt wird, kann die Vollständigkeit des Lösevorgangs besonders leicht beobachtet werden.

Es ist jedoch auch möglich, Lösungen der Komponenten der Formel (I) und (II) in einem geeigneten organischen Lösungsmittel, zum Beispiel Aceton, Chloroform oder Methanol, zu mischen und das Lösungsmittel nach gründlicher Durchmischung wieder zu entfernen, beispielsweise durch Destillation unter vermindertem Druck. Selbstverständlich muß bei dieser Verfahrensweise darauf geachtet werden, daß durch das Lösungsmittel keine Verunreinigungen oder unerwünschten Dotierungsstoffe eingeschleppt werden.

Durch geeignete Zusätze können die flüssigkristallinen Dielektrika nach der Erfindung so modifiziert werden, daß sie in allen bischer bekannt gewordenen Arten von Flüssigkristallanzeigeelementen verwendet werden können. Derartige Zusätze sind dem Fachmann bekannt und sind in der einschlägigen Literatur ausführlich beschrieben. Beispielsweise können dichroitische Farbstoffe oder Substanzen zur Veränderung der dielektrischen Anisotropie, der Viskosität, der Leitfähigkeit und/oder der Orientierung der nematischen Phasen zugesetzt werden. Derartige Substanzen sind zum Beispiel in den DE-OS 22 09 127, 22 40 864, 23 21 632, 23 38 281, 24 50 088, 26 27 430, 28 53 728 und 29 02 177 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz in Grad Celsius ; Siedetemperaturen sind mit Kp. bezeichnet. Wenn nichts anderes angegeben ist, bedeuten Angaben von Teilen oder Prozent Gewichtsteile bzw. Gewichtsprozent.

## Beispiel 1

a) 15,3 g 4-(trans-4-n-Pentylcyclohexyl)-biphenyl werden portionsweise in eine 40° warme Mischung aus 5 ml 65 % Salpetersäure und 6 ml 96 % Schwefelsäure eingetragen. Nach Ende der Zugabe wird das Reaktionsgemisch noch 1 Stunde bei 60° gerührt und auf 150 g Eis gegossen. Nach dem Auftauen wird das auskristallisierte 4-(trans-4-n-Pentylcyclohexyl)-4'-nitrobiphenyl abfiltriert und aus Ethanol umkristallisiert ; F. 115°, K. 176°.

b) In eine Suspension von 3 g Palladium-Kohle (10 % Pd) in einer Lösung von 10 g 4-(trans-4-n-Pentylcyclohexyl)-4'-nitrobiphenyl in 100 ml Tetrahydrofuran wird eine Stunde lang bei Normaldruck und

Raumtemperatur Wasserstoff eingeleitet. Anschließend wird vom Katalysator abfiltriert und das Filtrat eingedampft. Das zurückbleibende 4-(trans-4-n-Pentylcyclohexyl)-4'-aminobiphenyl wird aus Petrolether (Siedebereich 40-60°) umkristallisiert ; F. 132°, K. 215°.

c) 7,8 g 4-(trans-4-n-Pentylcyclohexyl)-4'-aminobiphenyl werden in 5 ml 36 % wäßriger Salzsäure aufgeschlämmt.

Nach Zugabe von 5 ml Dioxan wird bei 0° die Lösung von 1,9 g Natriumnitrit in 7,5 ml Wasser zugetropft. Unmittelbar darauf wird, ebenfalls bei 0°, eine Lösung von 6 g Natriumtetrafluorborat in 10 ml Wasser zugetropft. Der sich bildende Niederschlag wird 1/2 Stunde nach erfolgter Zugabe der Fluorverbindung abgesaugt, mit Eiswasser gewaschen und bei Raumtemperatur im Vakuum getrocknet. Das getrocknete Pulver wird auf 120° erhitzt. Nach Beendigung der $BF_3$-Entwicklung wird der Rückstand in Ethanol gelöst. Aus der Lösung kristallisieren 3,5 g 4-(trans-4-n-Pentylcyclohexyl)-4'-fluorbiphenyl, F. 99°, K. 154°.

Analog werden hergestellt :

4-(trans-4-Methylcyclohexyl)-4'-fluorbiphenyl,
4-(trans-4-Ethylcyclohexyl)-4'-fluorbiphenyl,
4-(trans-4-n-Propylcyclohexyl)-4'-fluorbiphenyl,
4-(trans-4-n-Butylcyclohexyl)-4'-fluorbiphenyl,
4-(trans-4-n-Hexylcyclohexyl)-4'-fluorbiphenyl,
4-(trans-4-n-Heptylcyclohexyl)-4'-fluorbiphenyl,
4-(trans-4-n-Decylcyclohexyl)-4'-fluorbiphenyl,
4-(trans-4-n-Dodecylcyclohexyl)-4'-fluorbiphenyl,
4-[trans-4-(2-Methylbutyl)-cyclohexyl]-4'-fluorbiphenyl.

Beispiel 2

a) 16,7 g 4-(trans-4-n-Pentylcyclohexyl)-4'-ethylbiphenyl werden in der in Beispiel 1 (a) beschriebenen Weise nitriert. Das nach dem Eingießen in Eiswasser abfiltrierte Gemisch isomerer Nitrierungsprodukte wird in Toluol gelöst und durch präparative Hochdruck-Flüssigchromatografie getrennt. Es werden 8,7 g 4-(trans-4-n-Pentylcyclohexyl)-2'-nitro-4'-ethylbiphenyl und 5,5 g 4-(trans-4-n-Pentylcyclohexyl)-2-nitro-4'-ethylbiphenyl erhalten.

b) Die nach dem Beispiel (2a) hergestellten Nitroverbindungen werden analog Beispiel (1b) zu den entsprechenden Aminoverbindungen reduziert und diese analog Beispiel (1c) in 4-(trans-4-n-Pentylcyclohexyl)-2'-fluor-4'-ethylbiphenyl, F. 27°, K. 104° und 4-(trans-4-n-Pentylcyclohexyl)-2-fluor-4'-ethylbiphenyl, F. 26°, K. 107°, überführt.

Analog werden hergestellt :

4-(trans-4-Ethylcyclohexyl)-2'-fluor-4'-n-butylbiphenyl,
4-(trans-4-n-Propylcyclohexyl)-2'-fluor-4'-n-propylbiphenyl,
4-(trans-4-n-Propylcyclohexyl)-2'-fluor-4'-n-octylbiphenyl,
4-(trans-4-n-Butylcyclohexyl)-2'-fluor-4'-n-butylbiphenyl,
4-(trans-4-n-Pentylcyclohexyl)-2'-fluor-4'-n-propylbiphenyl,
4-(trans-4-n-Pentylcyclohexyl)-2'-fluor-4'-n-pentylbiphenyl, Ubergang smektisch-nematisch 52°, K. 105° ;
4-(trans-4-n-Heptylcyclohexyl)-2'-fluor-4'-n-butylbiphenyl,
4-(trans-4-n-Octylcyclohexyl)-2'-fluor-4'-methylbiphenyl, und
4-(trans-4-Ethylcyclohexyl)-2-fluor-4'-n-butylbiphenyl,
4-(trans-4-n-Propylcyclohexyl)-2-fluor-4'-n-propylbiphenyl,
4-(trans-4-n-Propylcyclohexyl)-2-fluor-4'-n-octylbiphenyl,
4-(trans-4-n-Butylcyclohexyl)-2-fluor-4'-n-butylbiphenyl,
4-(trans-4-n-Pentylcyclohexyl)-2-fluor-4'-n-propylbiphenyl,
4-(trans-4-n-Pentylcyclohexyl)-2-fluor-4'-n-pentylbiphenyl,
4-(trans-4-n-Heptylcyclohexyl)-2-fluor-4'-n-butylbiphenyl,
4-(trans-4-n-Octylcyclohexyl)-2-fluor-4'-methylbiphenyl,
4-(trans-4-n-Pentylcyclohexyl)-2,2'-difluor-4'-ethylbiphenyl, F. − 2°, K. 80°.

Die folgenden Beispiele betreffen flüssigkristalline Dielektrika nach der Erfindung :

Beispiel A

Das flüssigkristalline Dielektrikum aus

24 % 4-(trans-4-n-Propylcyclohexyl)-benzonitril,
36 % 4-(trans-4-n-Pentylcyclohexyl)-benzonitril,
25 % 4-(trans-4-n-Heptylcyclohexyl)-benzonitril und
15 % 4-(trans-4-n-Pentylcyclohexyl)-biphenyl-4'-carbonitril

hat einen nematischen Bereich von − 6° bis + 70° und eine Viskosität von $28 \cdot 10^{-3}$ Pa.s bei 20° und von $97 \cdot 10^{-3}$ Pa.s bei 0°. Wird darin die zuletzt genannte Komponente durch die gleiche Gewichtsmenge 4-(trans-4-n-Pentylcyclohexyl)-4'-fluorbiphenyl ersetzt, so ändert sich der Temperaturbereich der nematischen Phase geringfügig auf von − 10° bis + 65° ; die Viskositätswerte, die einen wesentlichen Einfluß auf die Schaltzeiten haben, betragen in dem so hergestellten erfindungsgemäßen Dielektrikum $24 \cdot 10^{-3}$ Pa.s bei 20° und nur $68 \cdot 10^{-3}$ Pa.s bei 0°

**Ansprüche** (für die Vertragsstaaten : CH, DE, FR, GB, IT, LI, NL)

1. Fluorhaltige Cyclohexylbiphenylderivate der Formel (I)

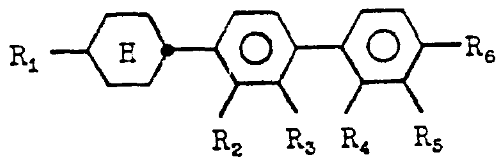

(I)

worin
$R_1$ Alkyl mit 1-12 C-Atomen,
$R_6$ Alkyl oder Alkoxy mit 1-12 C-Atomen, CN oder Fluor, und
$R_2$, $R_3$, $R_4$ und $R_5$ Wasserstoff oder Fluor bedeuten, mit der Maßgabe, daß mindestens einer, jedoch nicht mehr als zwei der Reste $R_2$, $R_3$, $R_4$, $R_5$ und $R_6$ Fluor bedeuten.

2. Verwendung der Cyclohexylbiphenylderivate der Formel (I) nach Anspruch 1 als Komponenten flüssigkristalliner Dielektrika.

3. Flüssigkristallines Dielektrikum mit zwei oder mehr flüssigkristallinen Komponenten, dadurch gekennzeichnet, daß mindestens eine Komponente ein Cyclohexylbiphenylderivat der Formel (I) nach Anspruch 1 ist.

4. Elektrooptisches Anzeigeelement auf der Basis einer Flüssigkristallzelle, dadurch gekennzeichnet, daß es ein flüssigkristallines Dielektrikum nach Anspruch 3 enthält.

**Ansprüche** (für den Vertragsstaat AT)

1. Verwendung fluorhaltiger Cyclohexylbiphenylderivate der Formel (I),

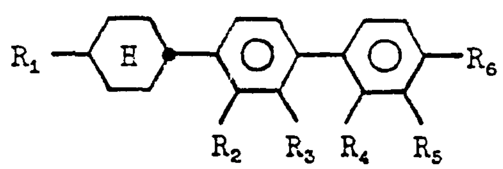

(I)

worin
$R_1$ Alkyl mit 1-12 C-Atomen,
$R_6$ Alkyl oder Alkoxy mit 1-12 C-Atomen, CN oder Fluor, und
$R_2$, $R_3$, $R_4$ und $R_5$ Wasserstoff oder Fluor bedeuten, mit der Maßgabe, daß mindestens einer, jedoch nicht mehr als zwei der Reste $R_2$, $R_3$, $R_4$, $R_5$ und $R_6$ Fluor bedeuten,
als Komponenten flüssigkristalliner Dielektrika.

2. Flüssigkristallines Dielektrikum mit zwei oder mehr flüssigkristallinen Komponenten, dadurch gekennzeichnet, daß mindestens eine Komponente ein Cyclohexylbiphenylderivat der Formel (I),

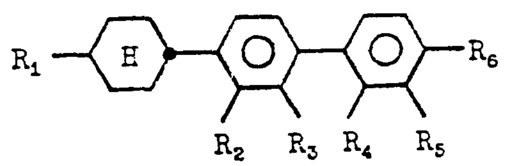

(I)

worin
$R_1$ Alkyl mit 1-12 C-Atomen,

$R_6$ Alkyl oder Alkoxy mit 1-12 C-Atomen, CN oder Fluor, und

$R_2$, $R_3$, $R_4$ und $R_5$ Wasserstoff oder Fluor bedeuten, mit der Maßgabe, daß mindestens einer, jedoch nicht mehr als zwei der Reste $R_2$, $R_3$, $R_4$, $R_5$ und $R_6$ Fluor bedeuten, ist.

3. Elektrooptisches Anzeigeelement auf der Basis einer Flüssigkristallzelle, dadurch gekennzeichnet, daß es ein flüssigkristallines Dielektrikum nach Anspruch 2 ist.

**Claims** (for the Contracting States : CH, DE, FR, GB, IT, LI, NL)

1. Cyclohexylbiphenyl derivatives of the formula (I)

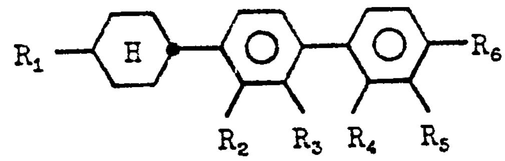

(I)

wherein

$R_1$ is alkyl having 1-12 C atoms,

$R_6$ is alkyl or alkoxy having 1-12 C atoms, CN or fluorine, and

$R_2$, $R_3$, $R_4$ and $R_5$ are hydrogen or fluorine, with the proviso that at least one, but not more than two of the radicals $R_2$, $R_3$, $R_4$, $R_5$ and $R_6$ are fluorine.

2. Use of the cyclohexylbiphenyl derivatives of the formula (I) according to Claim 1 as components of liquid-crystalline dielectrics.

3. Liquid-crystalline dielectric with two or more liquid-crystalline components, characterised in that at least one component is a cyclohexylbiphenyl derivative of the formula (I) according to Claim 1.

4. Electro-optical display element based on a liquid crystal cell, characterised in that it contains a liquid-crystalline dielectric according to Claim 3.

**Claims** (for the Contracting State AT)

1. Use of the Cyclohexylbiphenyl derivatives of the formula (I)

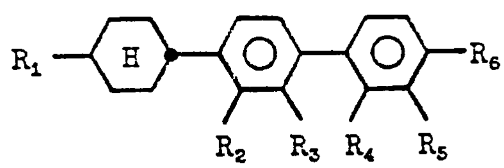

(I)

wherein

$R_1$ is alkyl having 1-12 C atoms,

$R_6$ is alkyl or alkoxy having 1-12 C atoms, CN or fluorine, and

$R_2$, $R_3$, $R_4$ and $R_5$ are hydrogen or fluorine, with the proviso that at least one, but not more than two of the radicals $R_2$, $R_3$, $R_4$, $R_5$ and $R_6$ are fluorine, as components of liquid-crystalline dielectrics.

2. Liquid-crystalline dielectric containing two or more liquid-crystalline components, characterised in that at least one component is a cyclohexylbiphenyl derivative of the formula (I)

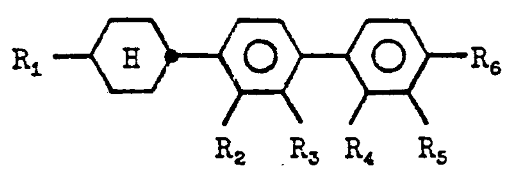

(I)

wherein

$R_1$ is alkyl having 1-12 C atoms,

$R_6$ is alkyl or alkoxy having 1-12 C atoms, CN or fluorine, and

$R_2$, $R_3$, $R_4$ and $R_5$ are hydrogen or fluorine, with the proviso that at least one, but not more than two of the radicals $R_2$, $R_3$, $R_4$, $R_5$ and $R_6$ are fluorine.

3. Electro-optical display element based on a liquid crystal cell, characterised in that it contains a liquid-crystalline dielectric according to Claim 2.

**Revendications** (pour les Etats contractants : CH, DE, FR, GB, IT, LI, NL)

1. Dérivés fluorés du cyclohexyl-biphényle, de formule I

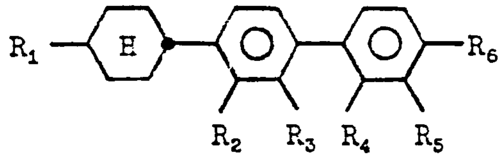

(I)

dans laquelle
$R_1$ représente un groupe alkyle en C 1-C 12,
$R_6$ représente un groupe alkyle ou alcoxy en C 1-C 12, un groupe CN ou le fluor, et
$R_2$, $R_3$, $R_4$ et $R_5$ représentent l'hydrogène ou le fluor, étant spécifié qu'au moins un et pas plus de deux des symboles $R_2$, $R_3$, $R_4$, $R_5$ et $R_6$ représentent le fluor.

2. Utilisation des dérivés du cyclohexylbiphényle de formule I selon la revendication 1, en tant que composants de diélectriques à cristaux liquides.

3. Diélectriques à cristaux liquides à deux ou plusieurs composants à cristaux liquides, caractérisé en ce qu'au moins un composant est un dérivé du cyclohexyl-biphényle de formule I selon la revendication 1.

4. Eléments indicateurs électro-optiques à base d'une cellule à cristaux liquides, caractérisés en ce qu'ils contiennent un diélectrique à cristaux liquides selon la revendication 3.

**Revendications** (pour l'Etat contractant AT)

1. Utilisation de dérivés fluorés du cyclohexyl-biphényle de formule I

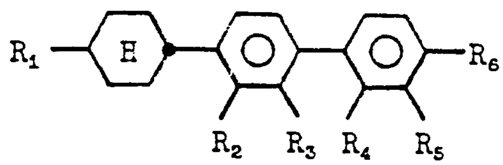

(I)

dans laquelle
$R_1$ représente un groupe alkyle en C 1-C 12,
$R_6$ représente un groupe alkyle ou alcoxy en C 1-C 12, un groupe CN ou le fluor, et
$R_2$, $R_3$, $R_4$ et $R_5$ représentent l'hydrogène ou le fluor, étant spécifié qu'au moins un mais pas plus de deux des symboles $R_2$, $R_3$, $R_4$, $R_5$ et $R_6$ représentent le fluor,
en tant que composants de diélectriques à cristaux liquides.

2. Diélectrique à cristaux liquides contenant deux ou plusieurs composants à cristaux liquides, caractérisé en ce qu'au moins un composant est un dérivé du cyclohexyl-biphényle de formule I

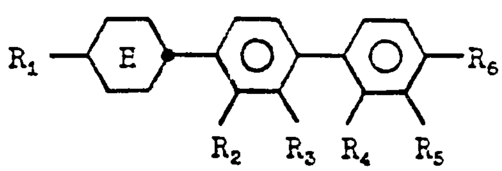

(I)

dans laquelle
$R_1$ représente un groupe alkyle en C 1-C 12,
$R_6$ représente un groupe alkyle ou alcoxy en C 1-C 12, un groupe CN ou le fluor, et
$R_2$, $R_3$, $R_4$ et $R_5$ représentent l'hydrogène ou le fluor, étant spécifié qu'au moins un mais pas plus de deux des symboles $R_2$, $R_3$, $R_4$, $R_5$ et $R_6$ représentent le fluor.

3. Elément indicateur électro-optique à base d'une cellule à cristaux liquides, caractérisé en ce qu'il consiste en un diélectrique à cristaux liquides selon la revendication 2.